# EUROPEAN PATENT SPECIFICATION

(11) **EP 3 860 663 B1**
(45) Date of publication and mention of the grant of the patent: **19.08.2026**
(21) Application number: 19868604.0
(22) Date of filing: 07.10.2019
(51) Int. Cl.: A61L 2/20, B28B 11/12, A61L 15/46, A61L 15/58, A61L 15/42

(54) **STERILIZATION OF MEDICAL DRESSINGS WITH ENHANCED ANTIMICROBIAL PROPERTIES**
STERILISATION VON MEDIZINISCHEN VERBÄNDEN MIT VERBESSERTEN ANTIMIKROBIELLEN EIGENSCHAFTEN
STÉRILISATION DE PANSEMENTS MÉDICAUX AUX PROPRIÉTÉS ANTIMICROBIENNES AMÉLIORÉES

(30) Priority: 05.10.2018 US 201862741839 P
(43) Date of publication of application: 11.08.2021
(73) Proprietor: Hydrofera, LLC, Manchester, CT 06042 (US)
(72) Inventor: DRURY, Thomas J., Narragansett, Rhode Island 02882 (US); HANSON, Christopher, East Hampton, Connecticut 06942 (US); MANTESE, John, Ellington, Connecticut 06029 (US); O'GARA, John E., Ashland, Massachusetts 01721 (US)
(74) Representative: Cohausz & Florack
(86) International application number: PCT/US2019/000051
(87) International publication number: WO 2020/072089

(56) References cited:
- WO-A1-2017/075320
- US-A- 5 811 471
- US-A- 5 908 693
- US-A- 5 908 693
- US-A1- 2014 018 654
- US-A1- 2014 018 654
- US-A1- 2014 275 864
- US-A1- 2014 275 864
- US-A1- 2015 352 244
- DATABASE Pubchem Compound 24 June 2005 (2005-06-24), "Gentian violet", XP055701667, retrieved from NCBI Database accession no. 11057
- A. RAZ-PASTEUR, EYTAN MAZOR, I. BERDICEVSKY, MAOZ SHEMESH, M. ZILBERMAN: "Effect of Gamma-irradiation Sterilization on the Antibacterial Efficacy and the Properties of a Hybrid Burn Dressing", JSM BURNS AND TRAUMA, 20 October 2016 (2016-10-20), pages 1 - 8, XP009527541, ISSN: 2475-9406

## Description

### RELATED APPLICATIONS

This application claims priority and benefits from provisional patent application No. 62/741,839 filed October 5, 2018.

### STATEMENT REGARDING FEDERALLY SPONSORED RESEARCH OR DEVELOPMENT

None

### REFERENCE TO SEQUENCE LISTING, A TABLE OR A COMPUTER PROGRAM LISTING COMPACT DISC APPENDIX

None.

### BACKGROUND OF THE INVENTION

### 1. Field of Invention

The present dressing relates to a method of sterilization of medical devices and more particularly, medical dressings. More particularly, the present invention relates to sterilization with gamma radiation and ethylene oxide of foamed polymers dressings containing the antimicrobial positive and negative dyes methylene blue and crystal violet and coated with a silicone adhesive.

### 2. Background of the Invention

The term sterilization refers to the elimination of microorganisms such as fungi, bacteria and viruses, or a reduction in the bioburden of an item where bioburden refers to the number of micro-organisms with which the item is contaminated. The degree of sterilization is typically measured by a sterility assurance level (SAL) which refers to the probability of a viable microorganism being present on a product unit after sterilization.

There are a number of sterilization procedures. The broad categories of sterilization include heat, chemicals, and irradiation. An example of using heat to sterilize is autoclaving of medical instruments. Cooking or canning food is also another application of using heat for sterilization. A number of chemicals can be used for sterilization including ozone, chlorine dioxide, ethylene oxide, and hydrogen peroxide. Irradiation includes exposure to gamma rays, x-rays, or an electron beam.

Many medical devices undergo terminal sterilization; that is sterilization occurs in the final packaged product. Thus, the sterilization operation may have a negative impact on the material of the device, and/or any active biocidal agents contained in the device. The present invention is directed to methods of sterilization that limit or eliminate some of these negative impacts of sterilization on the effectiveness of device, and particularly methods involving ethylene oxide sterilization.

Ethylene oxide (EO, EtO) sterilization is a broadly used terminal sterilization method for medical devices.

EO sterilization and irradiation as a terminal sterilization can also cause limitations for medical devices or more specifically wound dressings containing antimicrobial positive and negative dyes. One example is a limitation on materials that can be used as different materials have their properties changed by sterilization. In the case of wound dressings, medical silicone adhesives and silicon rubbers are excluded from use in gamma terminally sterile products, e.g. as cover dressings, despite their excellent clinical performance. Therefore, a wound dressing comprised of an antimicrobial positive and negative dyed wound dressing and a silicone adhesive is not known in the prior art.

In U.S. Patent Application Publication Number US2013/0032967 published February 7, 2013 describes a method of EO sterilization on polymeric stents where the temperature is kept below 40°C to limit or eliminate negative impacts on polymeric stents caused by ethylene oxide sterilization.

Hsiao et al. (Polymer Degradation and Stability, 2012, 97, 715) reported EO sterilization to have a substantial effect on the release pattern of drugs embedded in a biodegradable polymer scaffold. In one case, the total drug-releasing period for the EO-treated samples was much shorter than that of untreated controls, and the overall amount of released antibiotic was less.

Chrzanowski et al. (Journal of Applied Polymer Science, 2014, 131, 40812) reported that EtO sterilization resulted in destruction of poly (1-lactide-co-glycolide) (PLGA) vascular prostheses, where radiation sterilization did not affect the material.

Polymeric based wound dressings made from e.g. polyvinyl alcohol (PVA) or polyurethane - polyether (PU) polymers and that contain antimicrobial positive and negative dyes are terminally sterilized using irradiation, e.g. Hydrofera^{®} Blue (Hydrofera, LLC), RTD^{®} Wound Dressing (Keneric Healthcare), NovaGran^{™} Blue Plus Dressing^{™} (Principle Business Enterprises, Inc.). In all known cases, the use of medical silicone adhesives is excluded from the device's design.

None of the aforementioned references attempt to increase the effect of antimicrobial positive and negative dyes or allow the uses of silicone adhesives with sterile product to prevent or preclude biofilm from forming on wounds.

Wounds often have multiple barriers to healing. Wound healing and infection is influenced by the relationship between the ability of bacteria to create a stable, prosperous community within a wound environment and the ability of the host to control the bacterial community. Since bacteria are rapidly able to form their own protective microenvironment (biofilm) following their attachment to a surface, the ability of the host to control these organisms is likely to decrease as the biofilm community matures. Within a stable biofilm community, interactions between aerobic and anaerobic bacteria are likely to increase their net pathogenic effect, enhancing their potential to cause infection and delay healing. Over the last few years, some have linked biofilm to chronic wounds. Microscopic evaluation or chronic wounds showed well organized biofilm with extracellular polymeric substance adhered around colony bacteria in at least 60% of the chronic wounds.Examples of dressings with antimicrobial dyes can be found in US 2014/018654 A1.

There is thus a need for new methods of making dressings and other medical devices with absorbed or impregnated therapeutic agents, more specifically antimicrobial positive and negative dyes, where the medical devices' performance is not impaired by an EO sterilization step. The above noted teachings do not aid in the resolution of a number of practical difficulties that are resolved by the present invention.

### SUMMARY OF THE INVENTION

The present invention describes sterile dressings for treating wounds as defined by claims 1-10 and processes for sterilizing such dressings as defined by claims 11-12 and processes of producing such dressings as defined by claims 13-14. Enhanced antimicrobial properties are achieved through the method of using gamma irradiation as a processing step on the dyed medical device prior to final assembly and terminal EO sterilization.

The invention allows for incorporation of non-gamma stable parts, e.g. silicones, in the final assembled product followed by terminal EO sterilization of the finished device. The gamma irradiation process step prior to the terminal EO sterilization surprisingly gives improved antimicrobial properties in comparison to the same device without the prior irradiative step. In a preferred embodiment, the device is an antibacterial dressing. The invention is directed to methods and processes for making these terminal EO sterile antimicrobial positive and negative dyed medical devices.

It is an object of the invention to make sterilized dressings and medical devices with integrated antimicrobial positive and negative dyes using silicone adhesives.

It is another object of the invention to make a sterilized antimicrobial positive and negative dye containing sponge dressing whereby the dye effectiveness against micro-organisms is enhanced by the sterilization process.

It is yet another object of the invention to provide an effective wound dressing comprising antimicrobial polymer for the treatment of chronic wounds that is capable of donating and absorbing moisture and exudate for optimal wound healing.

### BRIEF DESCRIPTION OF THE DRAWINGS

The present invention will be described with reference to the appended Figures, in which:
Figure 1 is a schematic block drawing of gamma radiation with subsequent EO sterilization;
Figure 2 is a schematic block drawing of gamma radiation with two comparative EO sterilization methods;
Figure 3 is a schematic block drawing like Figure 2 with EO sterilization before the gamma sterilization step; and
Figure 4 is a plan view of the inventive dressing showing the silicone sheet extending beyond the sponge material.

These and other objects, advantages, and novel features of the present invention will become apparent when considered with the teachings contained in the detailed disclosure along with the accompanying drawings.

### DESCRIPTION OF THE INVENTION

Use of the singular herein includes the plural and vice versa unless expressly stated to be otherwise, or obvious from the context that such is not intended. That is, "a" and "the" refer to one or more of whatever the word modifies. For example, "a device" includes one device, two devices, etc. Likewise, "a polymer" may refer to one, two or more polymers, and "the polymer" may mean one polymer or a plurality of polymers. By the same token, words such as, without limitation, "devices "and "polymers" would refer to one device or polymer as well as to a plurality of devices or polymers unless, again, it is expressly stated or obvious from the context that such is not intended.

In the same manner, any ranges presented are inclusive of the end-points. For example, "a temperature between 10° C and 30° C" or "a temperature from 10° C to 30° C" includes 10° C and 30° C, as well as any temperature in between.

Also, words of approximation when used herein such as, without limitation, "about" "substantially," "essentially" and "approximately" mean that the word or phrase modified by the term need not be exactly that which is written but may vary from that written description to some extent. The extent to which the description may vary will depend on how great a change can be instituted and have one of ordinary skill in the art recognize the modified version as still having the properties, characteristics and capabilities of the modified word or phrase. In general, but with the preceding discussion in mind, a numerical value herein that is modified by a word of approximation may vary from the stated value by plus or minus 15%, unless expressly stated otherwise.

The aspects of the present invention are described in the following paragraphs along with their preferred embodiments. In the below text the term "wound" is to be understood in its broadest sense, i.e. as any exterior part of a human or animal body that may be in need of treatment, particularly antibacterial treatment. Examples of wounds in the present context includes but are not limited to: Any laceration to the skin, such as a wound, a chronic wound, a burn wound, a cut, wounds associated with dermatological conditions, grafts, pressure wounds, traumatic wounds, underlying infections with fistulation from bone, joint or soft tissue. The present invention uses polymeric foam or sponges treated with antibacterial or antimicrobial material which is placed over the wound.

### Wound Dressing Materials

The wound dressing may be a foam or sponge material and may be polymeric in composition where the polymer can be a synthetic substance, a natural substance or combinations thereof. In one example the synthetic polymer can be polyvinyl formal (PVF), polyvinyl acetal, polyurethane, polyester, polyethylene terephthalate or a mixture of polymers. The natural polymer material can be either animal or plant derived, for example, collagen, or chitosan. The preferred sponge material is polyvinyl acetal,
polyurethane or polyvinyl formal.

The foam or sponge has a morphology characterized by an average pore throat diameter of 0.5 - 500 µm, a fluid retention of 5.5 - 25.0 mL fluids/g porous material, a density of 0.05 - 0.15 g polymer/cm³ porous material, and a porosity of 60 - 99.5%. The cell structure is characterized as open / interconnected with through pores that can be evaluated by techniques such as capillary flow porometry and liquid extrusion porosimetry.

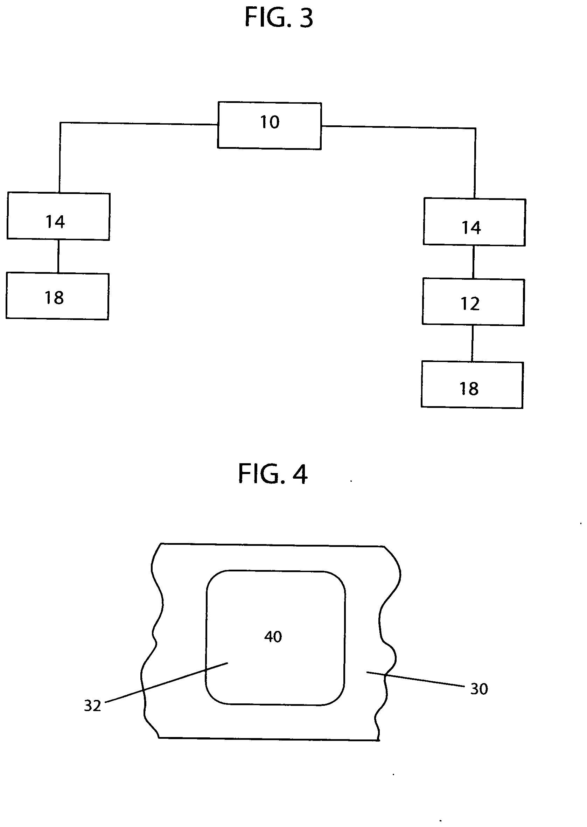

The silicon adhesive is attached to a silicone film sheet 30 and is applied to a planar surface 32 of the sponge material dressing 40. The film sheet may extend beyond the dressing where it can adhere to the skin surface and keep the dressing in place as is shown in Figure 4.

While the preferred embodiment of the invention is directed toward treated sponge dressings, it is envisioned as being used on other medical devices such as polyurethane catheters which are highly prone to infection.

### Dyes & Antimicrobial Agents

The dyes are clinically safe and have antimicrobial properties. The term "antimicrobial" is defined as having the ability to destroy or inhibit the growth of microorganisms, and comprises one or more of the following: antibacterial, antifungal, antiprotozoal, and antiviral. The inventive sterile dressing for treating wounds is made from a polymer sponge containing a plurality of antimicrobial dyes with at least one dye being gram positive and at least one other dye being gram negative. The dressing is exposed to both gamma radiation and ethylene oxide, which increase the sterilibility and allow a silicon adhesive to be secured to said sponge surface.

Dyes can include, for example, triaryl or diarylmethanes, methylene blue, toluidine blue, methylene violet, azure A, azure B, azure C, brilliant cresol blue, thionin, methylene green, bromcresol green, crystal violet, acridine orange, brilliant green, acridine yellow, quinacrine, trypan blue, trypan red and mixtures of these dyes. In the current invention the dyes are methylene blue and crystal violet. The above dye listing contains gram positive and gram negative dyes. Dyes of both charges are utilized to combat a large family of bacteria having gram positive and gram negative characteristics. Other antimicrobials agents can include e.g. chlorhexidine, rifampicin, sparfloxacin and triclosan.

### Radiation Activation after Terminal Sterilization

In another embodiment, the activation by irradiation occurs after terminal sterilization with EO.

In the case of devices with parts that are not compatible with the radiation type, masking techniques may be used to shield the non-compatible parts of the sterile device from radiation exposure.

In another irradiation method for devices with parts that are not compatible with the radiation type, radiation sources with spatial coherence (e.g. lasers) that allows for selectively activating areas of the device that are stable to radiation exposure. These radiation sources may also have high temporal coherence.

There is currently a need for effective medical products that include active substances which inhibit the growth of and/or kill bacteria, in particular there is a need for wound care products and methods that inhibit the growth of and/or kill biofilm forming bacteria more efficiently.

### Example 1

### Antibacterial Activity Test

Antibacterial activity is measured in the following manner.
1. Prepare duplicate samples of the dyed and processed sponge dressings material in 5 X 5 cm swatches.
2. Prepare duplicate samples in 5 x 5 cm swatches of the undyed sponge dressings as controls.
3. Inoculate each sample (treated and control) with 5.0 mL of approximately 1.0E+06 CFU/mL of each specified challenge organism.
4. Challenge organisms are: staphylococcus aureus (SA, ATCC# 6538), Escherichia coli (EC, ATCC# 11775), and pseudomonas aeruginosa (PA, ATCC# 9027). These bacteria are three of the five most common bacteria found in wound infections with staphylococcus aureus (SA, ATCC# 6538) being the most common.
5. Incubate samples in a humidified atmosphere for 24 hours at 37 °C.
6. After this initial incubation period express the fluid from each sample, and plate aliquots of serial dilutions to Tryptic-Soy Agar and re-incubate for at least an additional 24 hours at 37 °C.
7. Following incubation, all plates are removed and the total colony forming units enumerated.
8. Assess each treated material versus the original inoculum level.

### Example 2

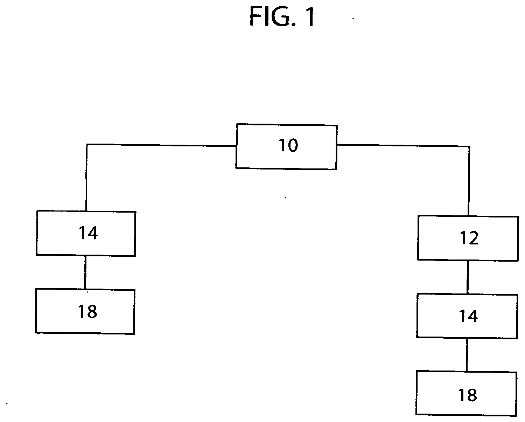

PU foam, PVA foam and PVF foam were dyed with methylene blue (MB) and crystal violet (CV), washed with water and dried. The dyed foams or sponge dressings 10 were then processed through gamma irradiation 12 and EO 14 steps to obtain test units 18 in the order shown in Figure 1.

### Gamma Irradiation

- 25 - 36 kilogray (kGy) dose achieved by controlled exposure to Cobalt 60 and EO Terminal Sterilization
- Pre-Conditioning: N/A
- Cycle Temperature: 108 ± 5°F
- Cycle Humidity: approximately 30% RH
- Sterilant (EO) Concentration: approximately 600 mg/L
- These EO process conditions represent the absolute minimum achievable set points that can reproducibly achieve a sterility assurance level (SAL) of 10⁻⁶ at a production scale.

After the terminal sterilization step, as shown in Figure 1, the dyed foam material samples were measured for antibacterial properties using the method described in Example 1, with the data being shown in Table 1. The data in Table 1 shows that gamma irradiation as a process step undertaken prior to EO sterilization gave better antibacterial activity than the EO sterilized only test samples.

**Table 1**

| Polymer | MB Load (mg/g) | CV Load (mg/g) | Process Gamma | Terminal EO | Average Log Reduction (CFU/mL) vs Inoculum | | |
|---|---|---|---|---|---|---|---|
| | | | | | SA | EC | PA |
| PVA | 0.50 | 0.80 | | X | 3.76 | 2.77 | 1.65 |
| PVA | 0.50 | 0.80 | X | X | 6.18 | 6.27 | 6.18 |
| PU | 0.08 | 0.60 | | X | 6.06 | 1.40 | 1.98 |
| PU | 0.08 | 0.60 | X | X | 6.18 | 6.27 | 6.18 |
| PU¹ | 0.49 | 0 | | X | -0.39 | -1.38 | -1.40 |
| PU¹ | 0.49 | 0 | X | X | 2.44 | -0.24 | 6.16 |
| PU | 0.27 | 0.35 | | X | 2.63 | -0.47 | 1.69 |
| PU | 0.27 | 0.35 | X | X | 3.73 | 6.20 | 6.16 |
| PU | 0.11 | 0.60 | | X | 2.04 | -0.61 | 6.14 |
| PU | 0.11 | 0.60 | X | X | 6.21 | 6.20 | 6.16 |
| PU | 0.00 | 0.55 | | X | 1.72 | -0.64 | 6.14 |
| PU | 0.00 | 0.55 | X | X | 6.21 | 6.20 | 6.16 |

The **Table 1 notation PU¹ in the Polymer column** denotes an example using only one dye.

This data shows surprising results with an increase in antibacterial dye effectiveness for both the PVA and PU sponges which is normally reduced during the EO sterilization step. This allows the use of silicone adhesive on the sponges treated with EO sterilization. As previously noted this Table includes data on polyurethane which includes single dye samples.

### Example 3

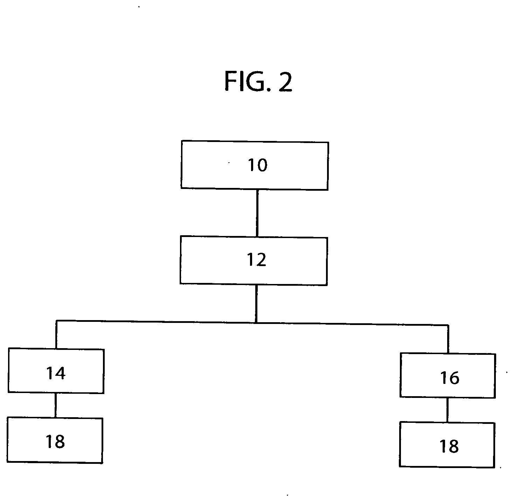

PU foam was dyed with methylene blue (MB) and crystal violet (CV), washed with water and dried. The dyed foam 10 was then processed using gamma irradiation 12 per Example 2 followed by EO 14 as described in Example 2 or by standard EO sterilization 16 conditions to obtain test samples 18 as listed below. The processing order is outlined in Figure 2.

### Standard Cycle EO Terminal Sterilization

- Dwell Time Limits: 24-48 hours, 50 - 80% RH, 40 - 52° C
- Cycle Temperature Range: 108 ± 5°F
- Cycle Humidity: < 55% RH
- Sterilant (EO) Concentration: approximately 600 mg/L

After the terminal step, the dyed foams were measured for antibacterial properties using the method described in Example 1. Antibacterial performance for the samples is found in Table 2.

**Table 2**

| Polymer | MB Load (mg/g) | CV Load (mg/g) | EO | EO | Average Log Reduction (CFU/mL) vs Inoculum | | |
|---|---|---|---|---|---|---|---|
| | | | Method Example 2 | Method Example 3 | SA | EC | PA |
| PU | 0.29 | 0.35 | X | | 6.27 | 6.24 | 6.25 |
| PU | 0.29 | 0.35 | | X | 6.23 | -0.78 | 6.21 |
| PU | 0.08 | 0.60 | X | | 6.27 | 6.24 | 6.25 |
| PU | 0.08 | 0.60 | | X | 6.23 | 0.20 | 6.21 |

### Example 4

PU foam was dyed with methylene blue (MB) and/or crystal violet (CV), washed with water and dried. The dyed foams 10 were then processed as noted in example 2 conditions with the EO 14 and/or gamma irradiative 12 steps in the order shown in Figure 3.

After the final step, the dyed foams were measured for antibacterial properties using the method described in Example 1, and the data is found in Table 3. The data shows that gamma irradiation 12 as a process step after EO sterilization gave better antibacterial activity than the EO sterilized only samples.

**Table 3**

| Polymer | MB Load (mg/g) | CV Load (mg/g) | Terminal EO | Process Gamma | Average Log Reduction (CFU/mL) vs Inoculum | | |
|---|---|---|---|---|---|---|---|
| | | | | | SA | EC | PA |
| PU | 0.08 | 0.60 | X | | 6.06 | 1.40 | 1.98 |
| PU | 0.08 | 0.60 | X | X | 3.00 | 6.20 | 6.13 |
| PU | 0.49 | 0 | X | | -0.39 | -1.38 | -1.40 |
| PU | 0.49 | 0 | X | X | 6.26 | 4.08 | 6.13 |
| PU | 0.27 | 0.35 | X | | 2.63 | -0.47 | 1.69 |
| PU | 0.27 | 0.35 | X | X | 6.26 | 6.26 | 6.13 |
| PU | 0.11 | 0.60 | X | | 2.04 | -0.61 | 6.14 |
| PU | 0.11 | 0.60 | X | X | 6.26 | 6.26 | 6.13 |
| PU | 0.00 | 0.55 | X | | 1.72 | -0.64 | 6.14 |
| PU | 0.00 | 0.55 | X | X | 6.26 | 6.26 | 3.92 |

### Example 5

Radiation Activation Prior to Dye Addition. White PU foam was processed using gamma radiation at a dose of 25-36 kGy. The foam was then dyed as described in Example 2 or 3 and sterilized by EO as described in Example 2 and 3 and the data found in the tables.

**Table 4**

| Polymer | MB Load (mg/g) | CV Load (mg/g) | EO Method | Process Gamma | Average Log Reduction (CFU/mL) vs Inoculum | | |
|---|---|---|---|---|---|---|---|
| | | | | | SA | EC | PA |
| PU | 0.29 | 0.35 | Ex2 X | X | 6.27 | 6.24 | 6,25 |
| PU | 0.29 | 0.35 | Ex3 X | X | 6.23 | -0.78 | 6.21 |

### Example 6

Thermal Characterization of Radiation Activated Polymers. Radiation activated polymers are characterized by a proportionally larger population of mobile polymer segments vs. their EO only sterilized analogs. Said population of more mobile segments may be distinguished from those without using differential scanning colorimetry, where differences in glass transition temperature (T_{g}) profiles are measured. In one example, the radiation activated polymers have one or more glass transition endotherms, and the EO only analogs have none detected. In another example, the radiation activated polymers show an increase of greater than 1° C in the T_{g} temperature in going from the 1st to 2^{nd} heat cycle vs the EO only analog which shows no change. Without wishing to be bound by theory, a change in the population of mobile polymer segments may translate to a more therapeutically effective antibacterial agent release from the material.

Dyed foam samples were processed through gamma irradiative and/or EO steps as described in Example 2. When both irradiation and EO steps were conducted, irradiation preceded EO.

Differential Scanning Calorimetry (DSC) was performed using a TA Q2000 Differential Scanning Calorimeter in combination with TA Universal Analysis software. At least 2 mg of each sample was placed in hermetically sealed pans for analysis. The samples were each analyzed in duplicate and the average value is reported in Table 5. Polyurethane samples were cooled to - 80 ° C, then heated to 200° C at 20 ° C/min, cooled back to -80 ° C at 20 ° C/min. and heated again to 200 ° C at 20 ° C/min. Polyvinyl alcohol samples were heated from 30 ° C to 90 ° C at 20 ° C/min, cooled back to 30 ° C at 20 ° C/min and heated again to 90 ° C at 20 ° C/min. Where more than one endotherm was observed, only the major endotherm is reported.

**Table 5**

| Dyed Polymer | Sample ID | Step 1 Gamma | Step 2 EO | Step 3 Gamma | (° C) | | |
|---|---|---|---|---|---|---|---|
| | | | | | 1st Heat | 2nd Heat | 1^{st} to 2nd |
| PVA | 042519.4 | X | | | 67.01¹ | 1.40 | 3.37 |
| PVA | 042519.5 | X | X | | 66.88 | 6.20 | 2.22 |
| PVA | 042519.6 | | X | | ND | ND | ND |
| PVA | 062619.1 | | X | X | 62.45² | 56.97 | 5.48 |
| PU | 042519.7 | X | | | -30.30 | -0.47 | 8.27 |
| PU | 042519.8 | X | X | | -31.07 | -28.18 | 2.89 |
| PU | 042519.9 | | X | | -31.81 | -31.81 | 0 |
| PU | 062619.2 | | X | X | -29.30 | -19.99 | 9.31 |

| | | | | | | | |
|---|---|---|---|---|---|---|---|
| **1.** A second minor endotherm observed on the first heat only at 48.56 ° C **2.** A second minor endotherm observed on the first heat only at 52.44 ° | | | | | | | |

### Biofilm Reduction Agents

Biofilm reduction agents are clinically safe and help remove biofilm by breaking up the film structure. Such agents are incorporated onto the sponge prior to the sterilization steps.

As an example, enzymes are used to target and break down the extracellular polymer substances of the biofilm and include amylase enzymes (e.g. amyloglucosidase, bacterial amylo novo), protease enzymes (e.g. savinase and everlase) fibrinolytic agents (e.g. plasmin, streptokinase, and nattokinase, and TrypLE), deoxyribonuclease I, glycoside hydrolase dispersin B, and cellulase.

Also chelating agents are used in the sponge body to sequester the metals that crosslink polysaccharides in the biofilm including ethylenediaminetetraacetic acid (EDTA), citrates, phosphonates and phosphonic acids.

Surfactants are used to solubilize the biofilm macromolecules, including ionic and non-ionic surfactants. Ionic surfactants may be negatively charged, positively charged, or zwitterionic, and include alkyl sulfates (e.g. sodium dodecyl sulfate), alkyl carboxylates, (e.g. sodium stearate), tertiary or quarternary alkyl ammoniums (e.g. cetrimonium bromide, cetrimonium chloride, cetrimonium stearate), 3-[(3-cholamidopropyl)dimethylammonio]-1-propanesulfonate (CHAPS). Non-ionic surfactants may include polyethylene glycol, polyethylene oxide, Triton, Tergitol, Pluronics, IGEPELS, Tweens, fatty acid esters.

## Claims

1. A sterile dressing for treating wounds comprised of a polymer sponge, containing a plurality of antimicrobial dyes with at least one dye being gram positive and at least one other dye being gram negative, wherein said gram positive and gram negative dyes are methylene blue and crystal violet respectively, said dressing being exposed to gamma radiation and sterilized by ethylene oxide, and a silicon adhesive secured to said sponge surface.

2. The sterile dressing of claim 1 wherein said sponge is taken from a group of polymers consisting of polyvinyl formal, polyvinyl acetal, polyurethane, polyester, or a mixture of said polymers, in particular wherein said polymer sponge is polyurethane or polyvinyl acetal.

3. The sterile dressing of claim 1 wherein said sponge has a morphology **characterized by** an average pore throat diameter of 0.5-500 µm, a fluid retention of 5.5-25.0 mL fluids/g porous material, a density of 0.05-0.15 g polymer/cm³ porous material, and a porosity of 60% to 99.5%.

4. The sterile dressing of claim 1 wherein said dressing has a cell structure which is open and interconnected by pores and wherein said cell structure can be evaluated by techniques such as capillary flow porometry and liquid extrusion porosimetry.

5. The sterile dressing of claim 1 wherein said sponge contains biofilm reducing agents taken from a group consisting of amylase enzymes (e.g. amyloglucosidase, bacterial amylo novo), protease enzymes (e.g. savinase and everlase) fibrinolytic agents (e.g. plasmin, streptokinase, and nattokinase, and TrypLE), deoxyribonuclease I, glycoside hydrolase dispersin B, and cellulase.

6. The sterile dressing of claim 1 wherein said sponge contains chelating agents to sequester the metals that crosslink polysaccharides in a biofilm, said chelating agents being taken from a group consisting of ethylenediaminetetraacetic acid (EDTA), citrates, phosphonates and phosphonic acids.

7. The sterile dressing of claim 1 wherein said sponge contains an ionic and non-ionic surfactant which are used to stabilize biofilm macromolecules.

8. The sterile dressing of claim 8 wherein said ionic surfactants are negatively charged, positively charged, or zwitterionic, and include alkyl sulfates (e.g. sodium dodecyl sulfate), alkyl carboxylates, (e.g. sodium stearate), tertiary or quarternary alkyl ammoniums (e.g. cetrimonium bromide, cetrimonium chloride, cetrimonium stearate), 3-[(3-cholamidopropyl)dimethylarnmoniol-1-propanesulfonate (CHAPS).

9. The sterile dressing of claim 1, said dressing being exposed to gamma radiation until it absorbs from about 5 to about 36 kilogray of radiation and sterilized by ethylene oxide;
said sponge having a morphology **characterized by** an average pore throat diameter of 0.5-500 gm and a porosity ranging from about 60% to about 99.5% and also containing at least one biofilm reducing agent, at least one chelating agent and an ionic and non-ionic surfactant.

10. The sterile dressing of claim 9 wherein said sponge contains biofilm reducing agents taken from a group consisting of amylase enzymes (e.g. amyloglucosidase, bacterial amylo novo), protease enzymes (e.g. savinase and everlase) fibrinolytic agents (e.g. plasmin, streptokinase, and nattolcinase, and TrypLE), deoxyribonuclease I, glycoside hydrolase dispersin B, and cellulose and/or wherein said sponge contains chelating agents to sequester the metals that crosslink polysaccharides in the biofilm taken from a group consisting of ethylenediaminetetraacetic acid (EDTA), citrates, phosphonates and phosphonic acids.

11. A process of sterilizing a dyed polymer sponge dressing of any one of claims 1-10 comprising the steps of:
a) dying a sponge dressing with antimicrobial dyes,
b) exposing the dyed sponge dressing to gamma radiation for a period suitable to apply gamma radiation ranging from about 5 to about 36 kilogray to said sponge dressing; and
c) treating the radiated sponge with an EO sterilization.

12. A process of sterilizing a dyed sponge dressing wherein said dyes are antimicrobial comprising the steps of:
a) dying a sponge with gram positive and gram negative dyes, wherein said gram positive and gram negative dyes are methylene blue and crystal violet respectively;
b) exposing the dyed sponge to gamma radiation; and
c) treating the radiated sponge with an EO sterilization having a cycle temperature of about 42.22 °C (108° F), a cycle humidity of about 30% RH and an EO sterilant concentration of about 600 mg/L.

13. A process for producing a sterile dressing for treating wounds according to any one of claims 1-10 comprising the sterilization of said dressing by both ethylene oxide and gamma radiation.

14. The process according to claim 13, wherein the polymer of the polymer dressing is taken from a group of polymers consisting of polyvinyl formal, polyvinyl acetal, polyurethane, and a mixture of said polymers.

## Patentansprüche

1. Ein steriler Verband zum Behandeln von Wunden, welcher einen Polymerschwamm umfasst, der eine Vielzahl von antimikrobiellen Farbstoffen enthält, wobei mindestens ein Farbstoff grampositiv und mindestens ein weiterer Farbstoff gramnegativ ist, wobei diese grampositiven und gramnegativen Farbstoffe Methylenblau und Kristallviolett sind, wobei dieser Verband Gammastrahlung ausgesetzt und mit Ethylenoxid sterilisiert ist und ein Siliconhaftmittel auf der Oberfläche dieses Schwamms aufgebracht ist.

2. Der sterile Verband nach Anspruch 1, wobei der Schwamm aus der Gruppe von Polymeren ausgewählt ist, die aus Polyvinylformal, Polyvinylacetal, Polyurethan, Polyester oder einem Gemisch aus diesen Polymeren besteht, insbesondere wobei dieser Polymerschwamm Polyurethan oder Polyvinylacetal ist.

3. Der sterile Verband nach Anspruch 1, wobei der Schwamm eine Morphologie besitzt, die durch einen mittleren Porenhalsdurchmesser von 0,5 bis 500 µm, eine Flüssigkeitsretention von 5,5 bis 25,0 ml Flüssigkeit/g porösem Material, eine Dichte von 0,05 bis 0,15 g polymer/cm³ porösem Material und eine Porosität von 60 % bis 99,5 % charakterisiert ist.

4. Der sterile Verband nach Anspruch 1, wobei dieser Verband eine Zellstruktur besitzt, die offen und durch Poren miteinander verbunden ist, und wobei diese Zellstruktur durch Verfahren wie Kapillarflussporometrie und Flüssigkeitsextrusionsporosimetrie beurteilt werden kann.

5. Der sterile Verband nach Anspruch 1, wobei der Schwamm Biofilm verringernde Mittel enthält, die aus der Gruppe ausgewählt sind, die aus Amylasen (beispielsweise Amyloglucosidase, Bacterial Amylo Novo), Proteasen (beispielsweise Savinase und Everlase), fibrinolytischen Wirkstoffe (beispielsweise Plasmin, Streptokinase und Nattokinase und TrypLE), Desoxyribonuclease I, Glykosidase Dispersin B und Cellulase besteht.

6. Der sterile Verband nach Anspruch 1, wobei der Schwamm Chelatisierungsmittel enthält, um die Metalle zu maskieren, welche Polysaccharide zu einem Biofilm vernetzen, wobei diese Chelatisierungsmittel aus der Gruppe ausgewählt sind, die aus Ethylendiamintetraessigsäure (EDTA), Citraten, Phosphonaten und Phosphonsäuren besteht.

7. Der sterile Verband nach Anspruch 1, wobei der Schwamm ein ionisches und ein nichtionisches Tensid enthält, die zur Stabilisierung von Biofilmmakromolekülen verwendet werden.

8. Der sterile Verband nach Anspruch 8, wobei die ionischen Tenside negativ geladen, positiv geladen oder zwitterionisch sind und Alkylsulfate (beispielsweise Natriumdodecylsulfat), Alkylcarboxylate (beispielsweise Natriumstearat), tertiäre oder quartäre Alkylammoniumverbindungen (beispielsweise Cetrimoniumbromid, Cetrimoniumchlorid, Cetrimoniumstearat) und 3-[(3-Cholamidopropyl)dimethylammonio]-1-propansulfonat (CHAPS) umfassen.

9. Der sterile Verband nach Anspruch 1, wobei dieser Verband Gammastrahlung so lange ausgesetzt wird, bis er etwa 5 bis etwa 36 kGray Strahlung absorbiert, und er mit Ethylenoxid sterilisiertwird, wobei der Schwamm eine Morphologie besitzt, die durch einen mittleren Porenhalsdurchmesser von 0,5 bis 500 µm und eine Porosität von etwa 60 % bis etwa 99,5 % charakterisiert ist, und auch mindestens ein Biofilm verringerndes Mittel, mindestens ein Chelatisierungsmittel und ein ionisches und ein nichtionisches Tensid enthält.

10. Der sterile Verband nach Anspruch 9, wobei der Schwamm Biofilm verringernde Mittel enthält, die aus der Gruppe ausgewählt sind, die aus Amylasen (beispielsweise Amyloglucosidase, Bacterial Amylo Novo), Proteasen (beispielsweise Savinase und Everlase), fibrinolytischen Wirkstoffe (beispielsweise Plasmin, Streptokinase und Nattokinase und TrypLE), Desoxyribonuclease I, Glykosidase Dispersin B und Cellulase besteht, und/oder wobei dieser Schwamm Chelatisierungsmittel enthält, welche, um die Metalle zu maskieren, die Polysaccharide zu einem Biofilm vernetzen, aus der Gruppe ausgewählt sind, die aus
Ethylendiamintetraessigsäure (EDTA), Citraten, Phosphonaten und Phosphonsäuren besteht.

11. Ein Verfahren zum Sterilisieren eines Verbandes aus gefärbtem Polymerschwamm nach einem der Ansprüche 1 bis 10, welches die Schritte:
a) Färben eines Schwammverbandes mit antimikrobiellen Farbstoffen,
b) Aussetzen des gefärbten Schwammverbandes einer Gammastrahlung einen Zeitraum lang, der für die Einwirkung einer Gammastrahlung von etwa 5 bis etwa 36 kGray auf diesen Schwammverband geeignet ist, und
c) Behandeln des bestrahlten Schwamms durch Sterilisieren mit EO
umfasst.

12. Ein Verfahren zum Sterilisieren eines Verbandes aus gefärbtem Schwamm, wobei die Farbstoffe antimikrobiell sind, welches die Schritte:
a) Färben eines Schwamms mit grampositiven und gramnegativen Farbstoffen, wobei diese grampositiven und gramnegativen Farbstoffe Methylenblau und Kristallviolett sind,
b) Aussetzen des gefärbten Schwamms einer Gammastrahlung und
c) Behandeln des bestrahlten Schwamms durch Sterilisieren mit EO mit einer Zyklustemperatur von etwa 42,22 °C (108°F), einer Zyklusluftfeuchtigkeit von etwa 30 % rF und einer Konzentration des Sterilisierungsmittels EO von etwa 600 mg/l
umfasst.

13. Ein Verfahren zur Herstellung eines sterilen Verbandes zum Behandeln von Wunden nach einem der Ansprüche 1 bis 10, welches das Sterilisieren dieses Verbandes mit sowohl Ethylenoxid als auch Gammastrahlung umfasst.

14. Das Verfahren nach Anspruch 13, wobei das Polymer des Polymerverbandes aus der Gruppe von Polymeren ausgewählt ist, die aus Polyvinylformal, Polyvinylacetal, Polyurethan und einem Gemisch aus diesen Polymeren besteht.

## Revendications

1. Pansement stérile pour le traitement de plaies constitué d'une éponge polymère, contenant une pluralité de colorants antimicrobiens avec au moins un colorant étant gram positif et au moins un autre colorant étant gram négatif, dans lequel lesdits colorants gram positif et gram négatif sont le bleu de méthylène et le violet cristallisé respectivement, ledit pansement étant exposé à un rayonnement gamma et stérilisé par de l'oxyde d'éthylène, et un adhésif de silicone fixé à ladite surface éponge.

2. Pansement stérile selon la revendication 1, dans lequel ladite éponge est tirée d'un groupe de polymères constitué de polyvinyl formal, d'acétal polyvinylique, de polyuréthane, de polyester, ou un mélange desdits polymères, en particulier dans lequel ladite éponge polymère est du polyuréthane ou de l'acétal polyvinylique.

3. Pansement stérile selon la revendication 1, dans lequel ladite éponge a une morphologie **caractérisée par** un diamètre moyen de gorge de pore de 0,5 à 500 µm, une rétention de fluide de 5,5 à 25,0 mL de fluides/g de matériau poreux, une masse volumique de 0,05 à 0,15 g de polymère/cm³ matériau poreux, et une porosité de 60 % à 99,5 %.

4. Pansement stérile selon la revendication 1, dans lequel ledit pansement a une structure cellulaire qui est ouverte et interconnectée par des pores, et dans lequel ladite structure cellulaire peut être évaluée par des techniques telles que la porométrie à flux capillaire et la porosimétrie par extrusion liquide.

5. Pansement stérile selon la revendication 1, dans lequel ladite éponge contient des agents réducteurs de biofilm pris dans un groupe constitué d'enzymes amylases (par exemple, amyloglucosidase, amylo novo bactérienne), d'enzymes protéases (par exemple, savinase et everlase), d'agents fibrinolytiques (par exemple, plasmine, streptokinase, et nattokinase, et TrypLE), de désoxyribonucléase I, de glycoside hydrolase dispersine B, et de cellulase.

6. Pansement stérile selon la revendication 1, dans lequel ladite éponge contient des agents chélateurs pour séquestrer les métaux qui réticule polysaccharides dans un biofilm, lesdits agents chélateurs étant pris dans un groupe constitué d'acide éthylènediaminetétraacétique (EDTA), de citrates, de phosphonates et d'acides phosphoniques.

7. Pansement stérile selon la revendication 1, dans lequel ladite éponge contient un agent tensioactif ionique et un agent tensioactif non ionique qui sont utilisés pour stabiliser des macromolécules de biofilm.

8. Pansement stérile selon la revendication 8, dans lequel lesdits agents tensioactifs ioniques sont chargés négativement, chargés positivement, ou zwitterioniques, et comportent des sulfates alkyliques (par exemple dodécyl sodique sulfate), des carboxylates alkyliques, (par exemple stéarate de sodium), des alkylammoniums tertiaires ou quaternaires (par exemple, bromure de cétrimonium, chlorure de cétrimonium, stéarate de cétrimonium), 3-[(3-cholamidopropyl)diméthylarnmoniol-1-propanesulfonate (CHAPS).

9. Pansement stérile selon la revendication 1, ledit pansement étant exposé à un rayonnement gamma jusqu'à ce qu'il absorbe d'environ 5 à environ 36 kilograys de rayonnement et stérilisé par de l'oxyde d'éthylène ;
ladite éponge ayant une morphologie **caractérisée par** un diamètre moyen de gorge de pore de 0,5 à 500 g et une porosité allant d'environ 60 % à environ 99,5 % et contenant également au moins un agent réducteur de biofilm, au moins un agent chélateur et un agent tensioactif ionique et non ionique.

10. Pansement stérile selon la revendication 9, dans lequel ladite éponge contient des agents réducteurs de biofilm pris dans un groupe constitué d'enzymes amylases (par exemple, amyloglucosidase, amylo novo bactérienne), d'enzymes protéases (par exemple, savinase et everlase), d'agents fibrinolytiques (par exemple, plasmine, streptokinase, et nattolcinase, et TrypLE), de désoxyribonucléase I, de glycoside hydrolase, de dispersine B, et de cellulose et/ou dans lequel ladite éponge contient des agents chélateurs pour séquestrer les métaux qui réticule polysaccharides dans le biofilm prélevé dans un groupe constitué d'acide éthylènediaminetétraacétique (EDTA), de citrates, de phosphonates et d'acides phosphoniques.

11. Procédé de stérilisation d'un pansement éponge polymère teint selon l'une quelconque des revendications 1 à 10, comprenant les étapes consistant à :
a) teindre un pansement éponge avec des colorants antimicrobiens,
b) exposer le pansement éponge teint à un rayonnement gamma pendant une période appropriée pour appliquer un rayonnement gamma allant d'environ 5 à environ 36 kilograys audit pansement éponge ; et
c) traiter l'éponge irradiée avec une stérilisation à base d'EO.

12. Procédé de stérilisation d'un pansement éponge teint dans lequel lesdits colorants sont antimicrobiens, comprenant les étapes consistant à :
a) teindre une éponge avec des colorants Gram positif et Gram négatif, dans lequel lesdits colorants Gram positif et Gram négatif sont respectivement le bleu de méthylène et le violet cristallisé ;
b) exposer l'éponge teinte à un rayonnement gamma ; et
c) traiter l'éponge irradiée avec une stérilisation EO ayant une température cyclique d'environ 42,22 °C (108 °F), une humidité cyclique d'environ 30 % HR et une concentration en EO stérilisant d'environ 600 mg/L.

13. Procédé de production d'un pansement stérile pour traiter des plaies selon l'une quelconque des revendications 1 à 10, comprenant la stérilisation dudit pansement à la fois par oxyde d'éthylène et par rayonnement gamma.

14. Procédé selon la revendication 13, dans lequel le polymère du pansement polymère est tiré d'un groupe de polymères constitué de polyvinyl formal, d'acétal polyvinylique, de polyuréthane, et un mélange desdits polymères.
